Europäisches Patentamt

European Patent Office

Office européen des brevets

(11) Publication number: **0 293 826**

**A2**

(12) # EUROPEAN PATENT APPLICATION

(21) Application number: 88108678.9

(51) Int. Cl.⁴: **A61K 31/725**

(22) Date of filing: **31.05.88**

(30) Priority: **02.06.87 US 57008**

(43) Date of publication of application:
**07.12.88 Bulletin 88/49**

(84) Designated Contracting States:
**AT BE CH DE ES FR GB GR IT LI LU NL SE**

(71) Applicant: **Stichting REGA V.Z.W.**
**Minderbroedersstraat 10**
**B-3000 Leuven(BE)**

(72) Inventor: **De Clercq, Erik Désire Alice**
**No. 35/7 Paul Lebrunstraat**
**B-3000 Leuven(BE)**
Inventor: **Ito, Masahiko**
**No. 74-1 Hichishamiya**
**Watari Fukushima 960(JP)**
Inventor: **Shigata, Shiro**
**No. 147-28 Kubouchi**
**Ohmori Fukushima 960-11(JP)**
Inventor: **Babe, Masanori**
**No. 47/3 Groot Begijnhof**
**B-3000 Leuven(BE)**

(74) Representative: **Prins, Hendrik Willem**
**Octrooibureau Arnold & Siedsma**
**Sweelinckplein, 1**
**NL-2517 GK The Hague(NL)**

(54) **Therapeutic and prophylactic application of sulfated polysaccharides against AIDS.**

(57) Sulfated polysaccharides, such as fucoidan, dextran sulfate, heparin and ⊤ι -, x- and λ-carrageenans proved to be potent and selective inhibitors of human immunodeficiency virus type 1 (HIV-1) in vitro. No toxicity for the host cells was noted with these compounds, so that the selectivity indexes, as based on the ratio of the 50% cytotoxic dose to the 50% antiviral effective dose, were equal to or in excess of 100.

Dextran sulfates of low molecular weight (5000, 8000) exhibited a significant inhibitory effect on HIV-1 at a concentration that was not markedly inhibitory to the blood coagulation process.

Figure 1

## Therapeutic and prophylactic application of sulfated polysaccharides against AIDS

This invention relates to the therapeutic and prophylactic application of sulfated polysaccharides for treating retroviral diseases, especially for treating AIDS and AIDS-related diseases. Examples of these sulfated polysaccharides are ι -, x- and λ-carrageenans.

The antiviral effects of the sulfated polysaccharides, dextran sulfate and heparin, have been reported in the early years of antiviral research (Takemoto, K.K. et al, (1965), Ann. N.Y. Acad. Sci. 130, 365-373; and Vaheri, A. (1964), Acta Pathol. Microbiol. Scand., Suppl. 171, 1-98). The polyanionic character of these compounds is reminiscent of other anionic compounds, such as suramin and Evans Blue, which contain six (suramin) or four (Evans Blue) sulfonic acid groups per molecule, and which have been shown previously to completely protect ATH8 cells against the cytopathogenicity of human immuno deficiency virus (HIV) at a concentration of 50 and 25 $\mu$g/ml, respectively (Balzarini, J., et al (1986), Biochem. Biophys. Res. Commun. 136, 64-71).

The pursuit of an effective antiviral chemotherapy for the acquired immune deficiency syndrome (AIDS) has yielded various candidates, i.e. suramin (Mitsuya, H. et al, (1984), Science 226, 172-174), phosphonoformate (Sandstrom, E.G. et al, (1985), Lancet i, 1480-1482), $3'$-azido-$2',3'$-dideoxythymidine (AZT) (Mitsuya, H. et al, (1985), Proc. Natl. Acad. Sci. USA 82, 7096-7100), and $2',3'$-dideoxynucleoside analogues (Mitsuya, H. et al, (1986), Proc. Natl. Acad. Sci. USA 83, 1911-1915), which show promise as inhibitors of human immunodeficiency virus (HIV), the causative agent of AIDS. As these compounds are assumed to act as inhibitors of the reverse transcriptase, and the latter is of pivotal importance in the replicative cycle of retroviruses, the inhibitory effects of the compounds on HIV replication can be readily accounted for (De Clercq, E. (1986), J. Med. Chem. 29, 1561-1569). Foremost among the compounds presently pursued as chemotherapeutic agents against AIDS is AZT which completely protects ATH8 cells against the cytopathogenicity of HIV at a concentration of 1-5 $\mu$M (Mitsuya, H. et al, (1985)), and has proven efficacious in prolonging the survival of AIDS and ARC (AIDS-related complex) patients (Mitsuya, H. et al, (1987), Nature 325, 773-778).

However, in view of the severity and complexity of AIDS and the toxicity problems associated with the clinical use of AZT, the search for effective anti-HIV agents should be intensified. Recently, we reported that glycyrrhizin (GL), one of the aqueous extracts of licorice root, is inhibitory to HIV replication in vitro (Ito, M. et al (1987), Antiviral Res. 7, 127-137). GL consists of one molecule of glycyrrhetinic acid (GA) and two molecules of glucuronic acid and, therefore, it shares some structural features with the carboxylate polyanions polyacrylic acid and polymethacrylic acid which are known to interfere with virus infectivity, at least partly through inhibition of virus adsorption (De Somer, P. et al, (1968), J. Virol. 2, 878.885); and De Somer, P. et al, (1968), J. Virol. 2, 886-893).

Further extending these observations we have now found that the sulfated polylsaccharides, such as dextran sulfate, heparin, fucoidan, and ι -, x- and λ -cannageenans are highly potent and selective inhibitors of HIV-replication in vitro. Their concentration values for complete protection of cells against the cytopathogenicity of HIV are comparable to those of suramin and Evans Blue, respectively. However, the selectivity indexes (ratio of $CD_{50}$ to $ED_{50}$) of dextran sulfate and heparin are much higher than those of suramin and Evans Blue, which are sulphonated aromatic compounds.

Dextran sulfates of low molecular weight (5000, 8000 Daltons) showed a significant inhibitory effect on HIV-I at a concentration that was not markedly inhibitory to the blood coagulation process.

MT-4 cells, Molt-4 (clone 8) cells and Molt-4/HTLV-III cells were kindly provided by the Department of Virology and Parasitology, Yamaguchi University School of Medicine, Japan.

The HTLV-I-carrying cell line, MT-4 (Harada, S. et al (1985), Virology 146, 272-281), and clone 8 of the human leukemic T-cell line Molt-4 (Kikukawa, R. et al, (1966), J. Virol. 57, 1159-1162), were used in our experiments. The cells were cultured and maintained in RPMI-1640 medium supplemented with 10% fetal calf serum (FCS), 100 IU/ml penicillin G and 100 $\mu$g/ml streptomycin (culture medium).

HIV was obtained from the culture supernatant of the Molt-4/HTLV-III cell line (Harada, S. et al (1985)).

Therapeutic and prophylactic compositions, containing as active ingredient a sulfated polysaccharide selected from the group consisting of dextran sulfate and heparin, for treating retroviral diseases, notably AIDS and AIDS-related diseases, and for administration prior to retroviral infection or prior to the onset of symptoms, to persons or patients at risk, respectively, in human practice may take the form of powders, suspensions, solutions, sprays, emulsions, unguents or creams and may be used for local application, for intranasal, rectal, vaginal and also for oral or parenteral (intravenous, intradermal, intramuscular, intrathecal etc.) administration. Such compositions may be prepared by combining (e.g. mixing, dissolving etc.) the active ingredients with pharmaceutically acceptable excipients of neutral character (such as aqueous or non-aqueous solvents, stabilizers, emulsifiers, detergents, additives), and further, if necessary with dyes and aromatizers. The concentration of the active ingredient in the therapeutic composition may vary widely between 0.1% and 100%, dependent on the mode of administration. Further, the dose of the active ingredient to be administered may vary between 1.0 mg and 100 mg per kg of body weight.

## EXAMPLE 1

### Inhibitory effect of dextran sulfate and heparin on the cytopathogenicity of HIV, after virus absorption

Dextran sulfate and heparin were evaluated for their inhibitory effect on the cytopathogenicity of HIV in MT-4 cells.

Dextran sulfate (MW (molecular weight): approximately 5000) and sodium heparin (197.1 units/ml) were obtained from Wako Chemical Co., Tokyo, Japan. Another preparation of dextran sulfate (MW: 5000) was obtained from Sigma Chemical Co., St. Louis, Missouri (USA). This preparation gave the same results as the preparation obtained from Wako Chemical Co. The compounds were dissolved in phosphate-buffered saline (PBS) and stored at $4^\circ$ C until used.

The inhibition of virus-induced cytopathogenicity, related to anti-viral activity of the tested compounds, was measured by trypan blue exclusion (Ito, M. et al, (1987)). MT-4 cells and Molt-4 (clone 8) cells were infected with HIV at a multiplicity of infection (MOI) of 0.002, and incubated for 1 hr at $37^\circ$ C. After virus adsorption, infected cells were washed and resuspended in culture medium. The number of MT-4 cells and Molt-4 (clone 8) cells was adjusted to $2 \times 10^5$ and $1 \times 10^5$ cells/ml, respectively. Then they were brought into each well of a flat bottomed 96-well plastic microtiter tray containing various concentrations of the test oompounds. After 4 days of incubation at $37^\circ$ C, half of the cells and culture medium were removed, and fresh medium containing the same concentration of the compounds was added. After another 2 days of incubation, the number of viable cells was determined microscopically in a hematocytometer by trypan blue exclusion.

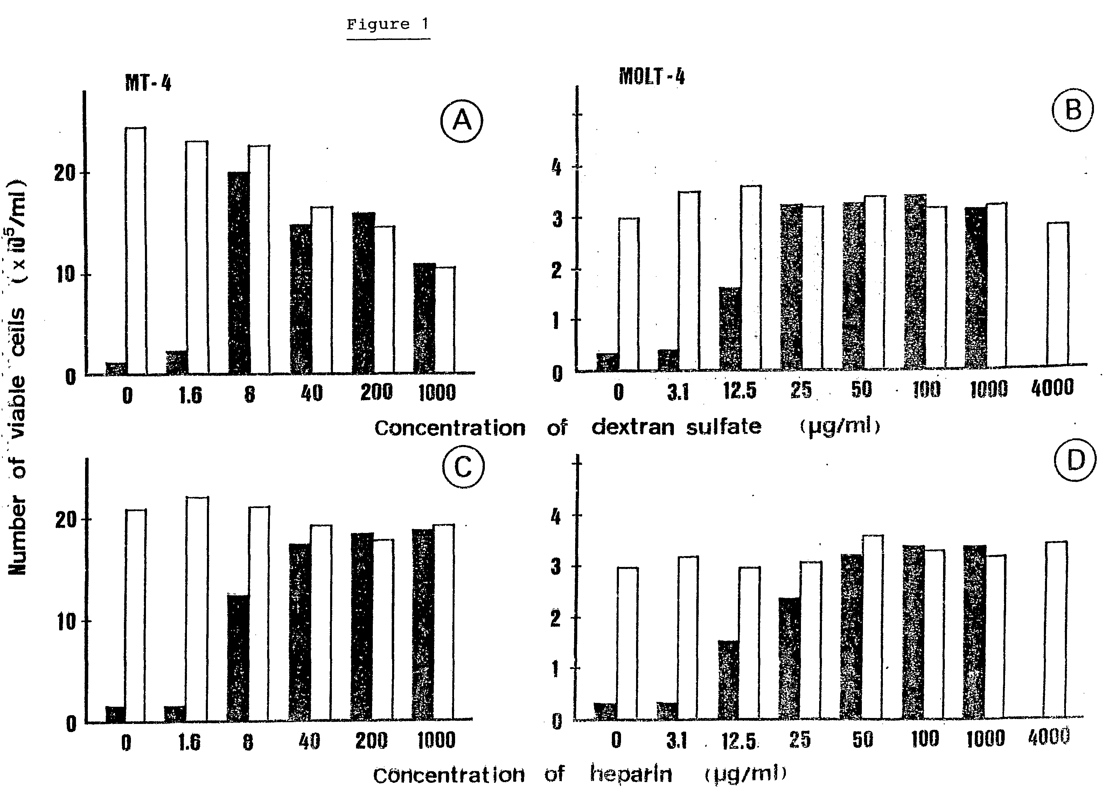

The results are shown in Table 1 and Figure 1.

Figure 1: is a representation of the test results on the inhibition of the cytogenicity of HIV for MT-4 and Molt-4 (clone 8) cells by dextran sulfate (A, B) or heparin (C, D). The cell viability was measured by trypan blue exclusion on day 6 after virus infection. The infected cells are indicated by solid columns (■) and the mock-infected cells are indicated by open columns (□).

TABLE 1 Inhibitory effects of dextran sulfate and heparin on the replication of HIV in MT-4 and Molt-4 (clone 8) cell cultures after virus absorption

| Compound | Cell line | ED$_{50}$[a]($\mu$g/ml) | CD$_{50}$[b]($\mu$g/ml) | SI[c] |
|---|---|---|---|---|
| Dextran sulfate | MT-4 | 4.7 | 470 | 100 |
| | Molt-4 | 14.1 | >4000 | >284 |
| Heparin | MT-4 | 7.5 | >1000 | >133 |
| | Molt-4 | 15.6 | >4000 | >256 |

a 50% Antiviral effective dose, based on the protection against the cytopathic effect of HIV.

b 50% Cytotoxic dose, based on a reduction in the viability of the mock-infected cells.

c Selectivity index (ratio of CD$_{50}$ to ED$_{50}$).

Dextran sulfate and heparin completely protected the cells against destruction by the virus at a concentration of 8 and 40 $\mu$g/ml, respectively (fig. 1A, 1C). When evaluated in Molt-4 (clone 8) cells, dextran sulfate and heparin achieved complete protection against HIV at a concentration of 25 and 50 $\mu$g/ml, respectively. (fig. 1B, 1D).

When assayed for cytotoxicity in mock-infected MT-4 cells and Molt-4 (clone 8) cells, dextran sulfate reduced MT-4 cell viability to 42% of control at a concentration of 1000 $\mu$g/ml, whereas it did not reduce the viability of Molt-4 (clone 8) cells at a concentration up to 4000 $\mu$g/ml (fig. 1A, 1B). Heparin did not affect the viability of either MT-4 cells or Molt-4 (clone 8) cells at a concentration up to 1000 and 4000 $\mu$g/ml, respectively (fig. 1C, 1D). The 50% antiviral effective dose (ED$_{50}$) of dextran sulfate and heparin for HIV-infected cells and their 50% cytotoxi city dose (CD$_{50}$) for mock-infected cells are presented in Table 1. The selectivity indexes (SI), as based on the ratio of CD$_{50}$ to ED$_{50}$, were 100 for dextran sulfate in MT-4 cells, >284 for dextran sulfate in Molt-4 cells, 133 for heparin in MT-4 cells and >256 for heparin in Molt-4 cells.

The selectivity indexes of dextran sulfate and heparin (>284 and >256, respectively in Molt-4 cells) are much higher than those of suramin and Evans Blue (5 and 4, respectively) (De Clercq, E. et al, (1986)).

The exact mechanism(s) of action of dextran sulfate and heparin against HIV replication remain to be elucidated. Dextran was found to be non-inhibitory to HIV replication (data not shown), which suggests that the anionic (sulfate) groups of dextran sulfate are necessary for its anti-HIV activity.

EXAMPLE 2

Inhibitory effect of several polysaccharides on the cytopathogenicity of HIV, during virus infection

Various polysaccharides were explored for their inhibitory effect on HIV-1 replication in vitro. When these compounds were evaluated for their inhibitory effects on the cytopathogenicity of HIV-1 in MT-4 cells, several sulfated oligo- and polysaccharides were found to be potent and selective inhibitors of HIV-1 replication.

MT-4 cells (input cell number: $3 \times 10^4$ cells well$^{-1}$) were cultured in microtray wells in the presence of various concentrations of the test compounds added immediately after infection with 100 CCID$_{50}$ of HIV-1 (CCID$_{50}$ being the 50% cell culture infective dose). After 5 days incubation at 37°C, the number of viable cells was determined by the MTT [3'-(4,5-dimethylthiazol-2-yl)-2,5-diphenyltetrazolium bromide] method, which is based on the formation of a blue formazan in living cells from a yellow tetrazolium dye. Antiviral activity and cytotoxicity of the test compounds are expressed as ED$_{50}$ and CD$_{50}$, which correspond to the doses required to reduce the number of viable cells in the virus- and mock-infected cell cultures by 50%, respectively. Anticoagulant activity was measured according to the clotting procedure for the quantitative determination of heparin in plasma (Sigma Technical Bulletin No. 870, Sigma Chemical Company, St. Louis, MO, U.S.A.) and is expressed in USP units.

The results are shown in Table 2.

## Table 2 Anti-HIV-1 activity and anticoagulant activity of polysaccharides

| Compound | ED$_{50}$ ($\mu$g ml$^{-1}$) | CD$_{50}$ ($\mu$g ml$^{-1}$) | Anticoagulant activity (U mg$^{-1}$) |
|---|---|---|---|
| Fucoidan | $1.4 \pm 0.43$ | $1060 \pm 210$ | 2.6 |
| Dextran sulfate (mw 5000) | $0.30 \pm 0.10$ | $>2500$ | 14.7 |
| Dextran (mw 90000) | $> 625$ | $>2500$ | 0.01 |
| Heparin (mw 11000) | $0.58 \pm 0.14$ | $>2500$ | 177 |
| N-desulfated heparin (mw 8800) | $> 625$ | $>2500$ | 0.6 |
| Dermatan sulfate | $>625$ | $>2500$ | 0.01 |
| Chondroitin sulfate | $230 \pm 14$ | $>2500$ | 0.5 |
| $\iota$-Carrageenan | $12 \pm 1.0$ | $> 625$ | 3.2 |
| $\kappa$-Carrageenan | $2.5 \pm 0.30$ | $> 625$ | 2.9 |
| $\lambda$-Carrageenan | $0.54 \pm 0.02$ | $> 625$ | 4.2 |

Potent and selective inhibitors of HIV-1 replication are fucoidan, dextran sulfate, heparin and $\iota$-, $\kappa$- and $\lambda$-carrangeenans. Dextran sulfate, $\lambda$-carrageenan and heparin also inhibited HIV-1 replication at very low concentrations, their ED$_{50}$ being 0.30, 0.54 and 0.58 $\mu$g ml$^{-1}$, respectively. Fucoidan and $\kappa$-carrageenan were 10-fold less active than pentosan polysulfate. Except for fucoidan the compound did not show any in vitro toxicity for the host MT-4 cells at the highest concentration assayed (2500 $\mu$g ml$^{-1}$ for pentosan polysulfate, dextran sulfate and heparin; 625$\mu$g ml$^{-1}$ for the $\iota$-, $\kappa$- and $\lambda$-carrageenans). For fucoidan, its 50% cytotoxic dose (CD$_{50}$) was about 1000 $\mu$g$^{-1}$.

In contrast, condroitin sulfate was only slightly active, whereas dextran, N-desulfated heparin and dermatan sulfate were totally inactive.

Since sulfated polysaccharides are known to interfere with the blood coagulation process, it was very important to compare their anticoagulant activities with their anti-HIV-1 effects in view of their potential clinical applicability in AIDS patients. As shown in Table 2, anticoagulant activity was directed for all compounds found active against HIV-1. The highest anticoagulant activity was achieved by heparin (177 U mg$^{-1}$). The anticoagulant effects of the other sulfated polysaccharides were more than 10 times lower than that of heparin. When the ED$_{50}$ values for anti-HIV-1 activity in MT-4 cells (Table 2) were converted from μg ml$^{-1}$ to U ml$^{-1}$, the following values were obtained: fucoidan, 3.6 x 10$^{-3}$ U ml$^{-1}$; τ -carrageenan, 3.8 x 10$^{-2}$ U ml$^{-1}$; x-carrageenan 7.3 x 10$^{-3}$ U ml$^{-1}$; λ-carrageenan, 2.3 x 10$^{-3}$ U ml$^{-1}$; dextran sulfate, 4.4 x 10$^{-3}$ U ml$^{-1}$; heparin, 1.0 x 10$^{-1}$ U ml$^{-1}$. Thus, except for heparin and τ -carrageenan, all compounds are active against HIV-1 at concentrations that are more than 100-fold lower than the anticoagulant threshold (1 U). In this respect, λ-carrageenans show the highest selectivity index namely 430.

## EXAMPLE 3

### Inhibitory effect of dextran sulfate and heparin on viral antigen expression

Dextran sulfate and heparin were evaluated for their inhibitory effect on viral antigen expression. Virus specific antigen expression in HIV-infectd MT-4 cells or Molt-4 (clone 8) cells was determined by indirect immunifluorescence (IF), using a polyclonal antibody from a seropostive anti-HIV human serum and fluorescein isothiocyanate (FITC)-conjugated rabbit anit-human IgG (Dakopatts A/S, Copenhagen, Denmark). More than 500 cells were counted under a fluorescent microscope and the percentage of fluorescent-positive cells was calculated.

The results of the inhibitory effect of dextran sulfate and heparin on the expression of HIV-specific antigens are shown in Table 3.

TABLE 3  Inhibitory effects of dextran sulfate and heparin on the expression of HIV-specific antigens in MT-4 and Molt-4 (clone 8) cells

| Concentration of compound (µg/ml) | Percentage of fluorescent MT-4 cells | |
|---|---|---|
| | dextran sulfate | heparin |
| 0 | 87.5 | 66.3 |
| 1.6 | 81.2 | 67.8 |
| 8 | 1.2 | 13.0 |
| 40 | 1.2 | 2.9 |
| 200 | 0.9 | 4.0 |
| 1000 | 2.7 | 2.1 |
| | Percentage of fluorescent Molt-4 cells | |
| 0 | 81.9 | 81.9 |
| 3.125 | 48.5 | 50.3 |
| 6.25 | 20.0 | 31.3 |
| 12.5 | 4.5 | 8.4 |
| 25 | 0.4 | 3.7 |
| 50 | 2.1 | 0.7 |
| 100 | 1.9 | 0.3 |
| 1000 | 0.2 | 0.6 |

Dextran sulfate and heparin achieved a complete inhibition of viral antigen expression in MT-4 cells at a concentration of 8 and 40 µg/ml, respectively, and in molt-4 (clone 8) cells at a concentration of 25 and 50 µg/ml, respectively. These concentrations were the same as those required for complete protection of the MT-4 and Molt-4 cells against HIV cytopathogenicity.

EXAMPLE 4

Inhibitory effect of dextran sulfate and dextran as function of their molecular weight on replication of HIV-1 in MT-4 cells

Dextran sulfates and dextrans of different molecular weight were explored for their inhibitory effect on replication of HIV-1.

Dextran (molecular weights (MW): approximately 90000 and 506000), dextran sulfate (MW: approximately 5000, 8000 and 500000), glucosamine-2-sulfate, -3-sulfate, -6-sulfate and -2,3-disulfate were purchased from Sigma Chemical Co., St. Louis, MO. Sodium heparin (mean MW: 15000) and fragmented heparins with varying molecular weights were kindly provided by Dr. Godtfredsen, Leo Pharmaceutical Products Ltd., Ballerup, Denmark. 2´,3´-Dideoxycytidine (DDC) was obtained from Pharmacia, Inc., Piscataway, NJ.

MT-4 cells were suspended at $5 \times 10^5$ cells per ml and infected with HIV-1 at 1000 $CCID_{50}$ per ml (1 $CCID_{50}$ being the 50% cell culture infective dose). After 2 h-incubation at 37° C, $5 \times 10^4$ cells per 100 $\mu$l were brought into each well of a flat-bottomed 96-well plastic microtiter tray containing 100 $\mu$l of various dilutions of the test compounds. After 5 days incubation at 37° C, the number of viable cells was determined microscopically in an hemacytometer by trypan blue exclusion. Anti-HIV-1 activity of the compounds was also determined by monitoring viral antigen expression in HUT-78 cells at day 14 after infection, measured by indirect immunifluorescence, using a polyclonal antibody as probe.

Dextran sulfates of different molecular weight (5000, 8000 and 50000) were equally effective in inhibiting HIV-1 replication in MT-4 cells. In contrast, dextran itself and glucosamine 2-, 3- or 6-sulfate or 2,3-disulfate did not show any anti-HIV-1 activity even at a concentration of 125 $\mu$g/ml (Table 4). Neither did these compounds show any cytotoxicity at a concentration up to 3125 $\mu$g/ml.

TABLE 4. Inhibitory effects of dextran sulfate and dextran
as function of their molecular weight, and heparin
on replication of HIV-1 in MT-4 cells

| Compound | IC$_{50}$ ($\mu$g/ml) | |
|---|---|---|
| | Antiviral activity* | Cytotoxicity* |
| Dextran sulfate | | |
| 5000 | 9.1 | > 3125 |
| 8000 | 10.7 | 1650 |
| 500000 | 10.9 | > 3125 |
| Dextran | | |
| 90000 ‡ | > 125 | > 3125 |
| 506000 | > 125 | > 3125 |
| Heparin | 7.0 | 1980 |

*Antiviral activity and cytotoxicity of the compounds expressed as the 50% inhibitory concentration (IC$_{50}$), required to reduce by 50% the number of viable cells in the virus- and mock-infected cell cultures, respectively.

‡Approximate molecular weight.

Data represent mean value for two separate experiments.

Since dextran sulfates show anti-coagulant activity, this activity was examined. Coagulation tests were performed manually on pooled normal plasma. For measuring the thrombin time, bovine thrombin was diluted so as to clot normal plasma in 20 seconds. For measuring the activated partial thromboplastin time (APTT), plasma was preincubated for 6 min with a mixture of Thrombofax® (a phospholipid mixture) and kaolin (a contact activator), after which coagulation was started by adding $CaCl_2$. The different dextran sulfates were diluted by Veronal buffer (pH 7.35) and added to plasma at a final concentration of 0, 2, 10, 50, 250, 2500 μg/ml. In all tests, a preincubation of 6 min at 37°C with the dextran sulfates was installed. Several controls were performed throughout the whole experiment and the clotting times were expressed as percent of the mean control.

The thrombin time was doubled with dextran sulfate (MW: 5000 or 8000) at a concentration of 50 μg/ml. Dextran sulfate (MW: 5000) caused a doubling of the APTT at a concentration of 20 μg/ml, whereas dextran sulfate (MW: 8000) did so at a concentration of 50 ug/ml. At 10μg/ml, which corresponds to the $IC_{50}$ for HIV-1 replication in MT-4 cells (Table 1), dextran sulfate (MW: 5000 or 8000) caused only a marginal prolongation of the APTT. However, dextran sulfate (MW: 500000) was the most patent anticoagulant, doubling the thrombin time and the APTT at a concentration of 2 μg/ml and 5 μg/ml, respectively.

Furthermore, dextran sulfate, heparin and other sulfated polysaccharides as well, may be subjected to various chemical modifications, i.e. "supersulfation" (Naggi, A., et al (1987), Biochem. Pharmacol., 36, 1895-1900), that may allow to dissociate antiretroviral from anticoagulant activity. Clinical trials carried out with intremittent intravenous injections of dextran sulfate have demonstrated that a plasma drug concentration of approximately 50 μg/ml required for doubling the clotting time, is easily attainabale in humans without critical side effects (Sasaki, S., et al (1964), Thrombosis Diathesis Haemorrhagica 12, 232-261). This concentration is considerably higher than the antivirally effective dose in vitro. When the compounds are supersulfated the optimal ratio for the number of sulfate groups pro sugar group appears to be unaverage 1-3, preferably 2.

Finally, dextran sulfate and heparin did not inhibit the proliferation of various cell lines, i.e. murine leukemia L1210, murine mammary FM3A, human B-lymphoblast Raji, human T-lymphoblast Molt/4F and human T4-lymphocyte CEM cells, at a concentration up to 5000 and 2500 ug/ml, respectively (data not shown). These results indicate that dextran sulfate and heparin are selective inhibitors of HIV-1 replication in vitro.

The above experimental results indicate that these sulfated polysaccharides are very valuable substrances which may block HIV replication in vivo and therefore are potential therapeutical and prophylactic agents for treating, preventing or inhibiting retroviral diseases, notably AIDS and AIDS-related diseases.

## Claims

1. A therapeutic or prophylactic composition for use in the treatment, prevention or inhibition of retroviral diseases, such as AIDS and AIDS-related diseases comprising as an active ingredient a sulfated polysaccharide selected and optionally a pharmaceutically acceptable excipient.

2. A therapeutic or prophylactic composition as claimed in claim 1, wherein said sulfated polysaccharide is selected from fucoidan, dextran sulfate, heparin and ʎ -, x-, and λ-carrageenans.

3. A therapeutic or prophylactic composition as claimed in claim 2, wherein dextran sulfate has a molecular weight of 1000 - 10.000, preferably 5000 - 8000 Daltons.

4. A therapeutic or prophylactic composition as claimed in claims 1-3, comprising said sulfated polysaccharide in a concentration ranging from 0.1 - 100% by weight.

5. A therapeutic or prophylactic composition as claimed in claim 1-4, having the form which is selected from the group consisting of powders, suspensions, solutions, sprays, emulsions, unguents and creams.

6. The use of a sulfated polysaccharide for the manufacture of a therapeutic or prophylactic composition for the treatment, prevention or inhibition of retroviral diseases such as AIDS and AIDS-related diseases.

7. The us as claimed in claim 6, where said sulfated polysaccharide is selected from fucoidan, dextran sulfate, heparin, and ʎ -, x-, and λ-carrageenans.

8. The use as claimed in claim 7, wherein dextran sulfate has a molecular weight of 1000 - 10.000, preferably 5000 - 8000 Daltons.

9. The use as claimed in claims 6-8, wherein said sulfated polysaccharide is in a concentration ranging from 0.1 - 100% by weight.

10. The use as claimed in claims 6-9 wherein the polysaccharide is persulfated.

11. The use as claimed in claim 10 wherein the ratio of the number of sulfate groups per associated sugar group is 2-3.

Figure 1

EP 0 293 826 A2